# EUROPÄISCHE PATENTSCHRIFT

(11) **EP 2 429 487 B1**
(45) Veröffentlichungstag und Bekanntmachung des Hinweises auf die Patenterteilung: **24.08.2016**
(21) Anmeldenummer: 10713334.0
(22) Anmeldetag: 09.04.2010
(51) Int. Cl.: A61K 8/81, A61Q 5/06, A61Q 5/12, A61K 8/04

(54) **POLYMERKOMBINATION AUS MINDESTENS ZWEI VERSCHIEDENEN N-VINYLPYRROLIDON / N-VINYLCAPROLACTAM-COPOLYMEREN FÜR GLANZGEBENDE KOSMETISCHE HAARBEHANDLUNGSMITTEL**
POLYMER COMBINATION OF AT LEAST TWO DIFFERENT N-VINYLPYRROLIDONE / N-VINYLCAPROLACTAM COPOLYMERS FOR COSMETIC HAIR SHINE PRODUCTS
ASSOCIATION DE POLYMÈRES COMPRENANT AU MOINS DEUX COPOLYMÈRES N-VINYLPYRROLIDONE / N-VINYLCAPROLACTAME DIFFÉRENTS POUR PRODUITS COSMÉTIQUES DE TRAITEMENT CAPILLAIRE DESTINÉS À DONNER DE LA BRILLANCE

(30) Priorität: 08.05.2009 DE 102009020553
(43) Veröffentlichungstag der Anmeldung: 21.03.2012
(73) Patentinhaber: Henkel AG & Co. KGaA, 40589 Düsseldorf (DE)
(72) Erfinder: SCHMARJE, Susanne, 22307 Hamburg (DE); BERGEMANN, Uwe, 22459 Hamburg (DE)
(86) Internationale Anmeldenummer: PCT/EP2010/054689
(87) Internationale Veröffentlichungsnummer: WO 2010/127923

(56) Entgegenhaltungen:
- EP-A1- 0 845 257
- EP-A1- 1 075 832
- EP-A2- 1 110 536
- WO-A1-96/19971
- DE-A1- 19 937 434
- US-A1- 2002 155 962
- PETTER P J ET AL: "Copolymer VC-713 - a new resin for hair spray formulation", SEIFEN, OELE, FETTE, WACHSE, AUGSBURG, DE, Bd. 113, Nr. 13, 1. Januar 1987 (1987-01-01), Seiten 427-430, XP009154316, ISSN: 0037-0983
- RAYMOND RIGOLETTO ET AL: "Polyquaternium-69: A New Fixative Polymer with Enhanced Styling Benefits", INTERNET CITATION, 1. Januar 2007 (2007-01-01), XP007910768, Gefunden im Internet: URL:http://www.cosmeticsciencetechnology.c om/articles/samples/1281.pdf [gefunden am 2009-12-03]

## Beschreibung

Die vorliegende Erfindung betrifft wasserarme Mittel zur Haarbehandlung in Form von Areosolsprays, enthaltend eine Polymerkombination aus mindestens zwei verschiedenen N-Vinylpyrrolidon/N-Vinylcaprolactam-Copolymeren und mindestens einen Alkohol sowie ein Verfahren zur Behandlung keratinhaltiger Fasern unter Applikation dieser Mittel als Aerosolspray. Unter keratinhaltigen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare.

Eine ansprechend aussehende Frisur wird heute allgemein als unverzichtbarer Teil eines gepflegten Äußeren angesehen. Dabei gelten aufgrund von aktuellen Modeströmungen immer wieder Frisuren als chic, die sich bei vielen Haartypen nur unter Verwendung festigender Wirkstoffe aufbauen bzw. für einen längeren Zeitraum bis hin zu mehreren Tagen aufrechterhalten lassen. Daher spielen Haarbehandlungsmittel, die einer permanenten oder temporären Formgebung der Haare dienen, eine wichtige Rolle. Temporäre Formgebungen, die einen guten Halt ergeben sollen, ohne das gesunde Aussehen der Haare, wie zum Beispiel deren Glanz, zu beeinträchtigen, können beispielsweise durch Haarsprays, Haarwachse, Haargele, Haarschäume, Fönwellen etc. erzielt werden.

Entsprechende Mittel zur temporären Formgebung enthalten als formgebende Komponente üblicherweise synthetische Polymere. Zubereitungen, die ein gelöstes oder dispergiertes Polymer enthalten, können mittels Treibgasen oder durch einen Pumpmechanismus auf das Haar aufgebracht werden. Insbesondere Haargele und Haarwachse werden allerdings in der Regel nicht direkt auf das Haar appliziert, sondern mittels eines Kamms oder der Hände im Haar verteilt.

Die zunächst wichtigste Eigenschaft eines Mittels zur temporären Verformung keratinischer Fasern, im Folgenden auch Stylingmittel genannt, besteht darin, den behandelten Fasern in der erzeugten Form einen möglichst starken Halt zu geben. Handelt es sich bei den keratinischen Fasern um menschliche Haare, spricht man auch von starkem Frisurenhalt oder vom hohen Haltegrad des Stylingmittels. Der Frisurenhalt wird im Wesentlichen durch die Art und Menge des eingesetzten synthetischen Polymers bestimmt, wobei jedoch auch ein Einfluss der weiteren Bestandteile des Stylingmittels gegeben sein kann.

Neben einem hohen Haltegrad müssen Stylingmittel eine ganze Reihe weiterer Anforderungen erfüllen. Diese können grob in Eigenschaften am Haar, Eigenschaften der jeweiligen Formulierung, z.B. Eigenschaften des Schaums, des Gels oder des versprühten Aerosols, und Eigenschaften, die die Handhabung des Stylingmittels betreffen, unterteilt werden, wobei den Eigenschaften am Haar besondere Wichtigkeit zukommt. Zu nennen sind insbesondere Feuchtebeständigkeit, niedrige Klebrigkeit und ein ausgewogener Konditioniereffekt. Weiterhin soll ein Stylingmittel möglichst für alle Haartypen universell einsetzbar sein.

Um den unterschiedlichen Anforderungen gerecht zu werden, wurde bereits eine Vielzahl von synthetischen Polymeren entwickelt, die in Stylingmitteln zur Anwendung kommen. Die Polymere lassen sich in kationische, anionische, nichtionische und amphotere filmbildende und/oder festigende Polymere unterteilen. Idealerweise ergeben die Polymere bei der Anwendung auf dem Haar einen Polymerfilm, der einerseits der Frisur einen starken Halt verleiht, andererseits aber hinreichend flexibel ist, um bei Beanspruchung nicht zu brechen. Ist der Polymerfilm zu brüchig, kommt es zur Bildung so genannter Filmplaken, das heißt Rückständen, die sich bei der Bewegung des Haares ablösen und den Eindruck vermitteln, der Anwender des entsprechenden Stylingmittels hätte Schuppen.

Weiterhin soll das temporär gestylte Haar neben dem starken Frisurenhalt gesund und natürlich aussehen. Dabei spielt der Haarglanz eine herausragende Rolle. Oftmals werden deshalb den Haarstylingmitteln sogenannte Glanzbildner in ausreichender Menge zugesetzt. Diese Glanzbildner sind beispielsweise Öle oder glanzgebende Pigmente wie z.B. Glimmerpartikel. Glanzgebende Partikel weisen den Nachteil auf, dass sie sich mit der Zeit vom Haar lösen und sich nach einiger Zeit z.B. auf der Kleidung oder der Gesichtshaut wieder finden. Öle beschweren das Haar und führen teilweise zu einer verschlechterten Haftung der filmbildenden bzw. festigenden Polymere auf dem Haar. Dies führt möglicherweise zu dem Nachteil, dass die aufgeprägte Frisur durch die filmbildenden bzw. festigenden Polymere nicht über eine ausreichende Dauer fixiert werden kann. Die Frisur hängt sich schneller aus.

In der europäischen Patentanmeldung EP 1 075 832 A1 werden wässrige Haarbehandlungsmittel, welche ein Terpolymer aus Vinylpyrrolidon, Vinylcaprolactam und einem basischen Acrylamidmonomer sowie mindestens einen weiteren, haarpflegenden kationaktiven Stoff enthalten, offenbart.

Aufgabe der vorliegenden Erfindung war es, ein Mittel zur temporären Verformung und/oder zur Pflege keratinischer Fasern zur Verfügung zu stellen, das sich durch einen hohen Haltegrad auszeichnet und einen hervorragenden Glanz auf den keratinhaltigen Fasern bewirken.

Es wurde nunmehr überraschenderweise gefunden, dass dies durch eine Kombination spezieller Polymere erreicht werden kann.

Ein erster Gegenstand der vorliegenden Erfindung ist daher ein Mittel zur Behandlung keratinhaltiger Fasern, insbesondere menschlichem Haar, enthaltend in einem kosmetisch akzeptablen Träger
(i) mindestens ein Copolymer (a) umfassend mindestens eine Struktureinheit der Formel (I) und mindestens eine Struktureinheit der Formel (II) und mindestens eine Struktureinheit der Formel (III) 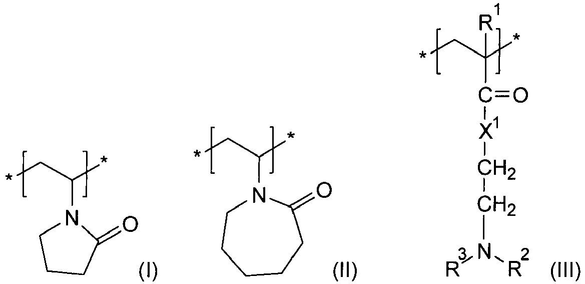 worin
   R¹ ein Wasserstoffatom oder eine Methylgruppe bedeutet,
   X¹ ein Sauerstoffatom oder eine Gruppe NH bedeutet,
   R² und R³ unabhängig voneinander für eine (C₁ bis C₄)-Alkylgruppe stehen, und
(ii) mindestens ein Copolymer (b) umfassend mindestens eine Struktureinheit der Formel (I) und mindestens eine Struktureinheit der Formel (II) und mindestens eine Struktureinheit der Formel (IV) 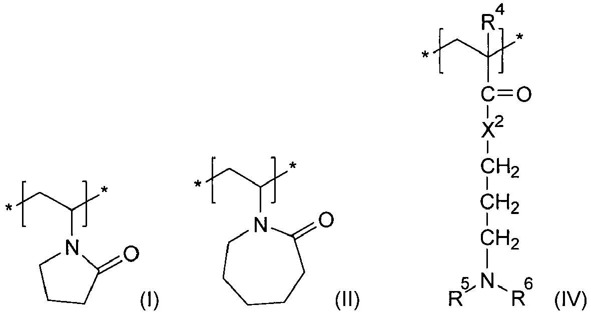 worin
   R⁴ ein Wasserstoffatom oder eine Methylgruppe bedeutet,
   X² ein Sauerstoffatom oder eine Gruppe NH bedeutet,
   R⁵ und R⁶ unabhängig voneinander für eine (C₁ bis C₄)-Alkylgruppe stehen und
(iii) weniger als 1 Gew.-% Wasser,
   wobei es zusätzlich mindestens einen Alkohol, der 2 bis 6 Kohlenstoffatome und 1 bis 3 Hydroxylgruppen aufweist, enthält,
   wobei es zusätzlich mindestens ein Treibmittel enthält und
   wobei es in Form eines Aerosolsprays vorliegt.
   Gemäß obiger Formeln und allen folgenden Formeln steht eine chemische Bindung, die mit dem Symbol * gekennzeichnet ist, für eine freie Valenz des entsprechenden Strukturfragments. Beispiele für (C₁ bis C₄)-Alkylgruppen gemäß Formel (III) und (IV) sind Methyl, Ethyl, Isopropyl, n-Propyl, n-Butyl, sec-Butyl, Isobutyl und tert-Butyl.

Erfindungsgemäß bevorzugte Mittel enthalten mindestens solch ein Copolymer (a), das gemäß Formel (III) mindestens einen der folgenden Parameter (besonders bevorzugt alle drei gemeinsam) erfüllt:
- R¹ steht für eine Methylgruppe,
- X¹ steht für ein Sauerstoffatom,
- R² und R³ stehen für eine Methylgruppe.

Ganz besonders bevorzugt enthält das erfindungsgemäße Mittel als Copolymer (a) ein Terpolymer aus N-Vinylpyrrolidon, N-Vinylcaprolactam und N,N-Dimethylaminoethylmethacrylat. Solche Copolymere können beispielsweise unter dem Handelnamen Advantage LC-E (INCI-Bezeichnung: Vinylcaprolactam/VP/Dimethylaminoethylmethacrylate Copolymer, Laurylpyrrolidone; 37 Gew.-% Aktivsubstanz in Ethanol mit Zusatz von N-Laurylpyrrolidon) oder Advantage LC-A (INCI-Bezeichnung: Vinylcaprolactam/VP/Dimethylaminoethylmethacrylate Copolymer; 37 Gew.-% Aktivsubstanz in Ethanol) von der Firma ISP bezogen werden.

Die Copolymere (a) sind erfindungsgemäß bevorzugt in dem erfindungsgemäßen Mittel in einer Menge von 1,0 Gew.-% bis 5 Gew.-%, besonders bevorzugt von 1,5 Gew.-% bis 3,0 Gew.-%, jeweils bezogen auf das Gewicht des kosmetischen Mittels, enthalten.

Erfindungsgemäß bevorzugte Mittel enthalten mindestens ein Copolymer (b), das gemäß Formel (IV) mindestens einen der folgenden Parameter (besonders bevorzugt alle drei gemeinsam) erfüllt:
- R⁴ steht für eine Methylgruppe,
- X² steht für eine Gruppe NH,
- R⁵ und R⁶ stehen für eine Methylgruppe.

Ganz besonders bevorzugt enthält das erfindungsgemäße Mittel als Copolymer (b) ein Terpolymer aus N-Vinylpyrrolidon, N-Vinylcaprolactam und N,N-Dimethylaminopropylmethacrylamid. Solche Copolymere sind beispielsweise unter dem Handelnamen Aquaflex SF 40 (INCI-Bezeichnung: VP/Vinyl Caprolactam/DMAPA Acrylates Copolymer, Alcohol Denat.; 38-42 Gew.-% Aktivsubstanz in Ethanol) bei der Firma ISP erhältlich.

Die Copolymere (b) sind erfindungsgemäß bevorzugt in dem erfindungsgemäßen Mittel in einer Menge von 0,5 Gew.-% bis 2,5 Gew.-%, insbesondere von 0,7 Gew.-% bis 1,5 Gew.-%, jeweils bezogen auf das Gewicht des kosmetischen Mittels, enthalten.

Darüber hinaus eignet sich ein Mischungsverhältnis von Copolymer (a) und Copolymer (b) in einem Gewichtsverhältnisbereich (i) zu (ii) von 1 zu 1 bis 1 zu 5, insbesondere von 1 zu 1.5 bis 1 zu 3, besonders bevorzugt zur Erzielung von maximalem Halt bei idealem Haarglanz.

Ein besonders bevorzugtes Mittel zur Behandlung keratinhaltiger Fasern, insbesondere menschlichem Haar, enthält in einem kosmetisch akzeptablen Träger
(i) mindestens ein Copolymer (a) umfassend mindestens eine Struktureinheit der Formel (I) und mindestens eine Struktureinheit der Formel (II) und mindestens eine Struktureinheit der Formel (III-1) 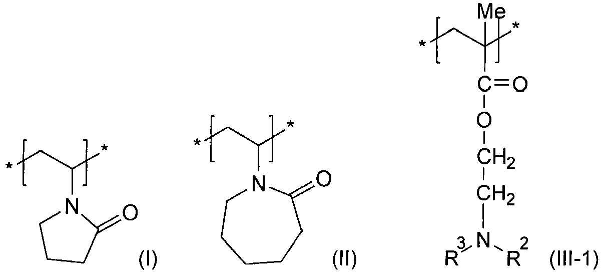 worin
   R² und R³ unabhängig voneinander für eine (C₁ bis C₄)-Alkylgruppe stehen,
   und
(ii) mindestens ein Copolymer (b) umfassend mindestens eine Struktureinheit der Formel (I) und mindestens eine Struktureinheit der Formel (II) und mindestens eine Struktureinheit der Formel (IV-1) 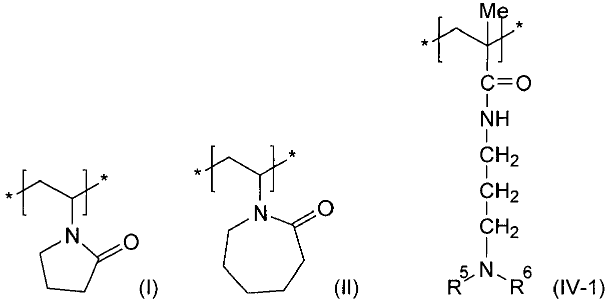 worin
   R⁵ und R⁶ unabhängig voneinander für eine (C₁ bis C₄)-Alkylgruppe stehen
   und
(iii) weniger als 1 Gew.-% Wasser,
   wobei es zusätzlich mindestens einen Alkohol, der 2 bis 6 Kohlenstoffatome und 1 bis 3 Hydroxylgruppen aufweist, enthält,
   wobei es zusätzlich mindestens ein Treibmittel enthält und
   wobei es in Form eines Aerosolsprays vorliegt.

Beispiele für (C₁ bis C₄)-Alkylgruppen gemäß Formel (III-1) und (IV-1) sind Methyl, Ethyl, Isopropyl, n-Propyl, n-Butyl, sec-Butyl, Isobutyl und tert-Butyl.

Ganz besonders bevorzugte erfindungsgemäß Mittel zur Behandlung keratinhaltiger Fasern, insbesondere menschlichem Haar, enthaltend in einem kosmetisch akzeptablen Träger
(i) als Copolymer (a) mindestens ein Terpolymer aus N-Vinylpyrrolidon, N-Vinylcaprolactam und N, N-Dimethylaminoethylmethacrylat
   und
(ii) als Copolymer (b) mindestens ein Terpolymer aus N-Vinylpyrrolidon, N-Vinylcaprolactam und N, N-Dimethylaminopropylmethacrylamid
   und
(iii) weniger als 1 Gew.-% Wasser,
wobei es zusätzlich mindestens einen Alkohol, der 2 bis 6 Kohlenstoffatome und 1 bis 3 Hydroxylgruppen aufweist, enthält,
wobei es zusätzlich mindestens ein Treibmittel enthält und
wobei es in Form eines Aerosolsprays vorliegt.

Das erfindungsgemäße Mittel enthält zwingend weniger als 1 Gew.-% Wasser bezogen auf das Gewicht des gesamten Mittels. Dabei ist es erfindungsgemäß umso bevorzugter, je weniger Wasser das erfindungsgemäße Mittel enthält. Erfindungsgemäß besonders bevorzugte Mittel enthalten daher Wasser in Mengen von weniger als 0,75 Gew.-%, insbesondere von weniger als 0,5 Gew.-%. Das erfindungsgemäße Mittel enthält die Inhaltsstoffe in einem kosmetisch akzeptablen Träger.

Dieser kosmetisch akzeptable Träger ist ein alkoholischer Träger. Erfindungsgemäß enthält das Mittel daher zusätzlich mindestens einen Alkohol, der 2 bis 6 Kohlenstoffatome und 1 bis 3 Hydroxylgruppen aufweist. Dieser zusätzliche Alkohol wird wiederum bevorzugt ausgewählt aus mindestens einer Verbindung der Gruppe, die gebildet wird aus Ethanol, Isopropanol, 1,2-Propylenglykol, Glyzerin, n-Butanol, 1,3-Butylenglykol. Ein ganz besonders bevorzugter Alkohol ist Ethanol.

Der zusätzliche Alkohol mit 2 bis 6 Kohlenstoffatomen und 1 bis 3 Hydroxylgruppen ist in dem erfindungsgemäß Mittel in Gegenwart mindestens eines Treibmittels bevorzugt in einer Menge von 40 Gew.-% bis 65 Gew.-%, insbesondere von 40 Gew.-% bis 50 Gew.-%, jeweils bezogen auf das Gewicht des kosmetischen Mittels, enthalten.

Als erfindungsgemäß geeignet erwies es sich, wenn das erfindungsgemäße Mittel optional zusätzlich mindestens ein N-(C₆ bis C₂₀)-Alkylpyrrolidon enthält. N-Laurylpyrrolidon ist ein erfindungsgemäß bevorzugt geeignetes N-(C₆ bis C₂₀)-Alkylpyrrolidon.

Die erfindungsgemäßen Effekte liessen sich durch Zusatz mindestens eines (C₂ bis C₆)-Trialkylcitrats zum erfindungsgemäßen Mittel steigern. Daher ist es erfindungsgemäß bevorzugt, wenn die Mittel zusätzlich mindestens eine Verbindung der Formel (E) enthalten, 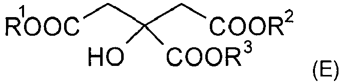 worin
R¹, R² und R³ unabhängig voneinander für eine (C₂ bis C₆)-Alkylgruppe stehen.

Beispiele einer (C₂ bis C₆)-Alkylgruppe gemäß Formel (E) sind Methyl, Ethyl, Isopropyl, n-Propyl, n-Butyl, sec-Butyl, Isobutyl, tert-Butyl, n-Pentyl, Neopentyl, Isopentyl, n-Hexyl.

Als besonders bevorzugte Verbindung der Formel (E) stellte sich Triethylcitrat heraus.

Das erfindungsgemäße Mittel enthält die Verbindungen der Formel (E) bevorzugt in einer Menge von 0,01 bis 1 Gew.-%, insbesondere von 0,05 bis 0,3 Gew.-%, jeweils bezogen auf das Gewicht des gesamten Mittels.

Eine ähnliche Steigerung der erfindungsgemäßen Effekte ließ sich durch Zusatz von Isopropylmyristat erzielen. Dieses ist in den erfindungsgemäßen Mitteln bevorzugt in einer Menge von 0,01 bis 1 Gew.-%, insbesondere von 0,05 bis 0,3 Gew.-%, jeweils bezogen auf das Gewicht des gesamten Mittels - enthalten.

Die erfindungsgemäßen Mittel können optional zusätzlich mindestens ein filmbildendes Polymer und/oder festigendes Polymer enthalten. Diese zusätzlichen Polymere sind von Copolymer (a) und Copolymer (b) verschieden. Die optional zugesetzten filmbildenden und/oder festigenden Polymere sind bevorzugterweise kationisch und/oder nichtionisch.

Unter filmbildenden Polymeren sind solche Polymere zu verstehen, welche beim Trocknen einen kontinuierlichen Film auf der Haut, dem Haar oder den Nägeln hinterlassen. Derartige Filmbildner können in den unterschiedlichsten kosmetischen Produkten wie beispielsweise Gesichtsmasken, Make-up, Haarfestigern, Haarsprays, Haargelen, Haarwachsen, Haarkuren, Shampoos oder Nagellacken verwendet werden. Bevorzugt sind insbesondere solche Polymere, die eine ausreichende Löslichkeit in Wasser oder Wasser/Alkohol-Gemischen besitzen, um in dem erfindungsgemäßen Mittel in vollständig gelöster Form vorzuliegen. Die filmbildenden Polymere können synthetischen oder natürlichen Ursprungs sein.

Unter filmbildenden Polymeren werden weiterhin erfindungsgemäß solche Polymere verstanden, die bei Anwendung in 0,01 bis 20 Gew.-%-iger wässriger, alkoholischer oder wässrigalkoholischer Lösung in der Lage sind, auf dem Haar einen transparenten Polymerfilm abzuscheiden.

Festigende Polymere tragen zum Halt und/oder zum Aufbau des Haarvolumens und der Haarfülle der Gesamtfrisur bei. Diese Polymere sind gleichzeitig auch filmbildende Polymere und daher generell typische Substanzen für formgebende Haarbehandlungsmittel wie Haarfestiger, Haarschäume, Haarwachse, Haarsprays. Die Filmbildung kann dabei durchaus punktuell sein und nur einige Fasern miteinander verbinden. Als eine Testmethode für die festigende Wirkung eines Polymers wird häufig der so genannte curl-retention - Test angewendet.

Zusätzlich kann das erfindungsgemäße Mittel mindestens ein filmbildendes kationisches und/oder festigendes kationisches Polymer enthalten.

Die zusätzlichen filmbildenden kationischen und/oder festigenden kationischen Polymere weisen mindestens eine Struktureinheit auf, die mindestens ein permanent kationisiertes Stichstoffatom enthält. Unter permanent kationisierten Stickstoffatomen sind solche Stickstoffatome zu verstehen, die eine positive Ladung tragen und dadurch eine quartäre Ammoniumverbindung bilden. Quartäre Ammonium-Verbindungen werden meist durch Umsetzung tertiärer Amine mit Alkylierungsmitteln, wie z.B. Methylchlorid, Benzylchlorid, Dimethylsulfat, Dodecylbromid, aber auch Ethylenoxid hergestellt. In Abhängigkeit von dem eingesetzten tertiären Amin sind insbesondere folgende Gruppen bekannt: Alkylammonium-Verbindungen, Alkenylammonium-Verbindungen, Imidazolinium-Verbindungen und Pyridinium-Verbindungen.

Im Sinne dieser Ausführungsform bevorzugte Mittel enthalten die filmbildenden kationischen und/oder festigenden kationischen Polymere in einer Menge von 0,1 Gew.-% bis 20,0 Gew.-%, besonders bevorzugt von 0,2 Gew.-% bis 10,0 Gew.-%, ganz besonders bevorzugt von 0,5 Gew.-% bis 5,0 Gew.-%, jeweils bezogen auf das Gewicht des Mittels.

Die kationischen filmbildenden und/oder kationischen festigenden Polymere können erfindungsgemäß aus kationischen, quaternisierten Cellulose-Derivaten ausgewählt werden.

Es erweisen sich generell solche kationischen, quaternisierten Cellulosen als im Sinne der Ausführungsform vorteilhaft, die in einer Seitenkette mehr als eine permanente kationische Ladung tragen. Darunter sind unter den kationischen Cellulosederivaten solche hervorzuheben, die aus Reaktion von Hydroxyethylcellulose mit einem Dimethyldiallylammonium-Reaktanden (insbesondere Dimethyldiallylammoniumchlorid) gegebenenfalls in Gegenwart weiterer Reaktanden hergestellt werden. Unter diesen kationischen Cellulosen sind wiederum solche kationischen Cellulosen mit der INCI-Bezeichnung Polyquaternium-4 besonders geeignet, welche beispielsweise unter den Bezeichnungen Celquat^{®} H 100, Celquat^{®} L 200 von der Firma National Starch vertrieben werden.

Weiterhin eignen sich solche kationischen filmbildenden und/oder kationischen festigenden Polymere, die mindestens eine Struktureinheit der Formel (I) und mindestens eine Struktureinheit der Formel (VI) und gegebenenfalls mindestens eine Struktureinheit der Formel (V) umfassen 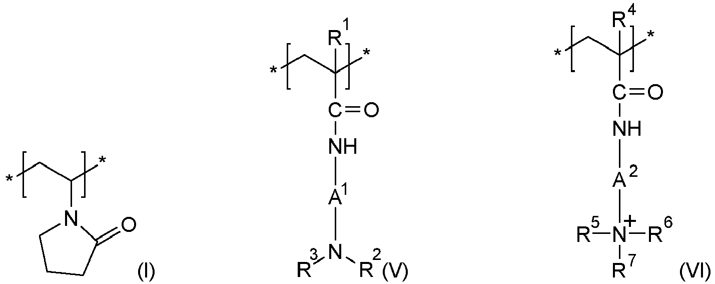 worin
R¹ und R⁴ stehen unabhängig voneinander für ein Wasserstoffatom oder eine Methylgruppe,
A¹ und A² stehen unabhängig voneinander für eine Gruppe Ethan-1,2-diyl, Propan-1,3-diyl oder Butan-1,4-diyl,
R², R³, R⁵ und R⁶ stehen unabhängig voneinander für eine (C₁ bis C₄)-Alkylgruppe,
R⁷ steht für eine (C₈ bis C₃₀)-Alkylgruppe.

Zur Kompensation der positiven Ladung des Monomers (VI) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Geeignete Verbindungen sind beispielsweise als
- Copolymere aus mit Diethylsulfat quaterniertem Dimethylaminoethylmethacrylat, mit Vinylpyrrolidon mit der INCI-Bezeichnung Polyquaternium-11 unter den Bezeichnungen Gafquat^{®} 440, Gafquat^{®}734, Gafquat^{®}755 (jeweils Firma ISP) sowie Luviquat PQ 11 PN (Firma BASF SE),
- Copolymere aus Methacryloylaminopropyllauryldimethylammonium chlorid mit Vinylpyrrolidon und Dimethylaminopropylmethacrylamid mit der INCI-Bezeichnung Polyquaternium-55 unter den Handelsnamen, Styleze^{®} W-10, Styleze^{®} W-20 (Firma ISP),
im Handel erhältlich.

Als im Sinne der Ausführungsform besonders bevorzugt verwendbare filmbildende und/oder festigende Polymere ausgewählt aus kationischen Polymeren die mindestens eine Struktureinheit enthalten, die ein permanent kationisiertes Stickstoffatom aufweisen, dienen weiterhin solche kationischen filmbildenden und/oder kationischen festigenden Copolymere, die mindestens ein Strukturelement der Formel (M1) enthalten 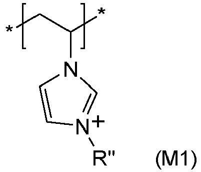 worin
R" für eine (C₁ bis C₄)-Alkylgruppe, insbesondere eine Methylgruppe, steht, und zusätzlich mindestens ein weiteres kationisches und/oder nichtionisches Strukturelement aufweisen.

Zur Kompensation der positiven Polymerladung gilt das oben gesagte.

Es ist wiederum im Rahmen dieser Ausführungsform erfindungsgemäß bevorzugt, wenn in dem erfindungsgemäßen als kationisches filmbildendes und/oder kationisches festigendes Polymer mindestens ein Copolymer (c1) enthalten ist, das neben mindestens einem Strukturelement der Formel (M1) zusätzlich ein Strukturelement der Formel (I) umfasst 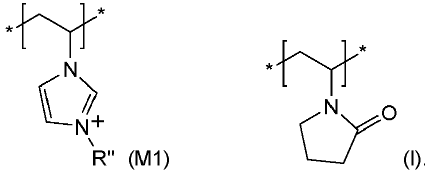 worin
R für eine (C₁ bis C₄)-Alkylgruppe, insbesondere eine Methylgruppe, steht.

Zur Kompensation der positiven Polymerladung der Copolymere (c1) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Ganz besonders bevorzugte kationische filmbildende und/oder kationische festigende Polymere als Copolymere (c1) enthalten 10 bis 30 Mol-%, vorzugsweise 15 bis 25 Mol.-% und insbesondere 20 Mol.-% Struktureinheiten gemäß Formel (M1) und 70 bis 90 Mol.-%, vorzugsweise 75 bis 85 Mol.-% und insbesondere 80 Mol.-% Struktureinheiten gemäß Formel (I).

Hierbei ist besonders bevorzugt, wenn die Copolymere (c1) neben Polymereinheiten, die aus dem Einbau der genannten Struktureinheiten gemäß Formel (M1) und (I) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (c1) ausschließlich aus Struktureinheiten der Formel (M1) mit R" = Methyl und (I) aufgebaut und lassen sich durch die allgemeine Formel (Poly1) 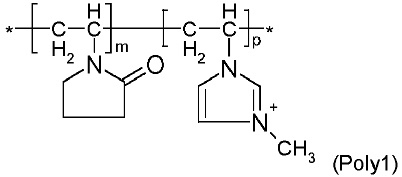 beschreiben, wobei die Indices m und p je nach Molmasse des Polymers variieren und nicht bedeuten sollen, daß es sich um Blockcopolymere handelt. Vielmehr können Struktureinheiten der Formel (M1) und der Formel (I) im Molekül statistisch verteilt vorliegen.

Wird in zur Kompensation der positiven Ladung des Polymers der Formel (Poly1) ein Chloridion verwendet, so werden diese N-Methylvinylimidazol/Vinylpyrrolidon-Copolymere werden laut INCI-Nomenklatur als Polyquaternium-16 bezeichnet und sind beispielsweise von der BASF unter den Handelsnamen Luviquat^{®} Style, Luviquat^{®} FC 370, Luviquat^{®} FC 550, Luviquat^{®} FC 905 und Luviquat^{®} HM 552

Wird in zur Kompensation der positiven Ladung des Polymers der Formel (Poly1) ein Methosulfat verwendet, so werden diese N-Methylvinylimidazol/Vinylpyrrolidon-Copolymere werden laut INCI-Nomenklatur als Polyquaternium-44 bezeichnet und sind beispielsweise von der BASF unter den Handelsnamen Luviquat^{®} UltraCare erhältlich.

Besonders bevorzugte erfindungsgemäße Mittel dieser Ausführungsform enthalten ein Copolymer (c1), insbesondere der Formel (Poly1), das Molmassen innerhalb eines bestimmten Bereiches aufweist. Hier sind erfindungsgemäße Mittel bevorzugt, bei denen das Copolymer (c1) eine Molmasse von 50 bis 400 kDa, vorzugsweise von 100 bis 300 kDa, weiter bevorzugt von 150 bis 250 kDa und insbesondere von 190 bis 210 kDa aufweist.

Zusätzlich zu dem bzw. den Copolymer(en) (c1) oder an dessen bzw. deren Stelle können die erfindungsgemäßen Mittel auch Copolymere (c2) enthalten, die ausgehend vom Copolymer (c1) als zusätzliche Struktureinheiten Struktureinheiten der Formel (II) aufweisen

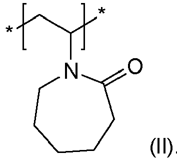

Weitere besonders bevorzugte erfindungsgemäße Mittel dieser Ausführungsform sind somit dadurch gekennzeichnet, daß sie als kationisches filmbildendes und/oder kationisches festigendes Polymer mindestens ein Copolymer (c2) enthalten, das mindestens eine Struktureinheit gemäß Formel (M1-a) und mindestens Struktureinheit gemäß Formel (I) und mindestens Struktureinheit gemäß Formel (II) enthält

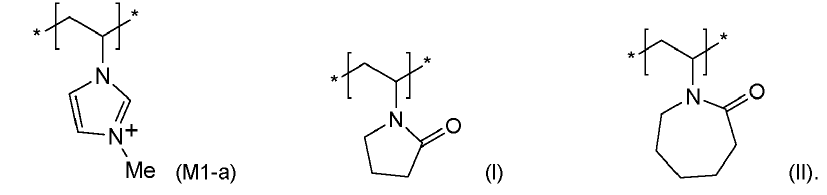

Auch hierbei ist es im Rahmen dieser Ausführungsform besonders bevorzugt, wenn die Copolymere (c2) neben Polymereinheiten, die aus dem Einbau der genannten Struktureinheiten gemäß Formel (M1-a), (I) und (II) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (c2) ausschließlich aus Struktureinheiten der Formeln (M1-a), (I) und (II) aufgebaut und lassen sich durch die allgemeine Formel (Poly2) 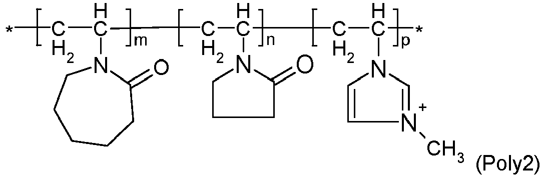 beschreiben, wobei die Indices m, n und p je nach Molmasse des Polymers variieren und nicht bedeuten sollen, daß es sich um Blockcopolymere handelt. Vielmehr können Struktureinheiten der besagten Formeln im Molekül statistisch verteilt vorliegen.

Zur Kompensation der positiven Polymerladung der Komponente (c2) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Wird in zur Kompensation der positiven Ladung des Polymer der Formel (Poly2) ein Methosulfat verwendet werden solche N-Methylvinylimidazol/Vinylpyrrolidon/Vinylcaprolactam-Copolymere laut INCI-Nomenklatur als Polyquarternium-46 bezeichnet und sind beispielsweise von der BASF unter dem Handelsnamen Luviquat^{®} Hold erhältlich.

Ganz besonders bevorzugte Copolymere (c2) enthalten 1 bis 20 Mol-%, vorzugsweise 5 bis 15 Mol.-% und insbesondere 10 Mol.-% Struktureinheiten gemäß Formel (M1-a) und 30 bis 50 Mol.-%, vorzugsweise 35 bis 45 Mol.-% und insbesondere 40 Mol.-% Struktureinheiten gemäß Formel (I) und 40 bis 60 Mol.-%, vorzugsweise 45 bis 55 Mol.-% und insbesondere 60 Mol.-% Struktureinheiten gemäß Formel (II).

Besonders bevorzugte erfindungsgemäße Mittel dieser Ausführungsform enthalten ein Copolymer (c2), das Molmassen innerhalb eines bestimmten Bereiches aufweist. Hier sind erfindungsgemäße Mittel bevorzugt, bei denen das Copolymer (c2) eine Molmasse von 100 bis 1000 kDa, vorzugsweise von 250 bis 900 kDa, weiter bevorzugt von 500 bis 850 kDa und insbesondere von 650 bis 710 kDa aufweist.

Zusätzlich zu dem bzw. den Copolymer(en) (c1) und/oder (c2) oder an dessen bzw. deren Stelle können die erfindungsgemäßen Mittel als filmbildendes kationische und/oder festigendes kationisches Polymer auch Copolymere (c3) enthalten, die als Struktureinheiten Struktureinheiten der Formeln (M1-a) und (I) aufweisen, sowie weitere Struktureinheiten aus der Gruppe der Vinylimidazol-Einheiten und weitere Struktureinheiten aus der Gruppe der Acrylamid- und/oder Methacrylamid-Einheiten.

Weitere besonders bevorzugte erfindungsgemäße Mittel dieser Ausführungsform sind dadurch gekennzeichnet, daß sie als zusätzliches kationisches filmbildendes und/oder kationisches festigendes Polymer mindestens ein Copolymer (c3) enthalten, das mindestens eine Struktureinheit gemäß Formel (M1-a) und mindestens weitere Struktureinheit gemäß Formel (I) und mindestens weitere Struktureinheit gemäß Formel (VII) enthält und mindestens weitere Struktureinheit gemäß Formel (VIII) enthält

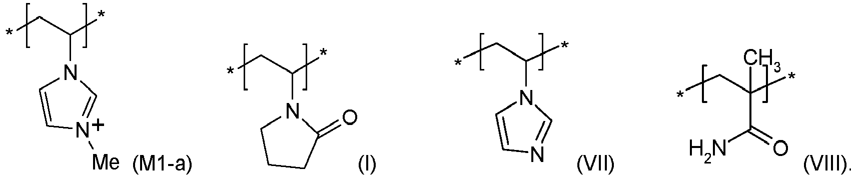

Auch hierbei ist es im Rahmen dieser Ausführungsform besonders bevorzugt, wenn die Copolymere (c3) neben Polymereinheiten, die aus dem Einbau der genannten Struktureinheiten gemäß Formel (M1-a), (I), (VII) und (VIII) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (c3) ausschließlich aus Struktureinheiten der Formel (M1-a), (I), (VII) und (VIII) aufgebaut und lassen sich durch die allgemeine Formel (Poly3) 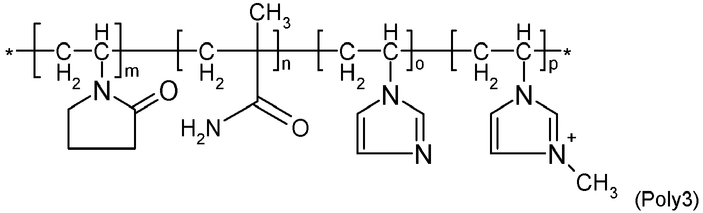 beschreiben, wobei die Indices m, n, o und p je nach Molmasse des Polymers variieren und nicht bedeuten sollen, daß es sich um Blockcopolymere handelt. Vielmehr können Struktureinheiten der Formeln (M1-a), (I), (VII) und (VIII) im Molekül statistisch verteilt vorliegen.

Zur Kompensation der positiven Polymerladung der Komponente (c2) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Wird in zur Kompensation der positiven Ladung des Polymer der Formel (Poly3) ein Methosulfat verwendet werden solche N-Methylvinylimidazol/Vinylpyrrolidon/Vinylimidazol/Methacrylamid-Copolymere laut INCI-Nomenklatur als Polyquaternium-68 bezeichnet und sind beispielsweise von der BASF unter dem Handelsnamen Luviquat^{®} Supreme erhältlich.

Ganz besonders bevorzugte Copolymere (c3) enthalten 1 bis 12 Mol-%, vorzugsweise 3 bis 9 Mol.-% und insbesondere 6 Mol.-% Struktureinheiten gemäß Formel (M1-a) und 45 bis 65 Mol.-%, vorzugsweise 50 bis 60 Mol.-% und insbesondere 55 Mol.-% Struktureinheiten gemäß Formel (I) und 1 bis 20 Mol.-%, vorzugsweise 5 bis 15 Mol.-% und insbesondere 10 Mol.-% Struktureinheiten gemäß Formel (VII) und 20 bis 40 Mol.-%, vorzugsweise 25 bis 35 Mol.-% und insbesondere 29 Mol.-% Struktureinheiten gemäß Formel (VIII).

Besonders bevorzugte erfindungsgemäße Mittel dieser Ausführungsform enthalten ein Copolymer (c3), das Molmassen innerhalb eines bestimmten Bereiches aufweist. Hier sind erfindungsgemäße Mittel bevorzugt, bei denen das Copolymer (c3) eine Molmasse von 100 bis 500 kDa, vorzugsweise von 150 bis 400 kDa, weiter bevorzugt von 250 bis 350 kDa und insbesondere von 290 bis 310 kDa aufweist.

Unter den zusätzlichen filmbildenden kationischen und/oder festigenden Polymer ausgewählt aus den kationischen Polymeren mit mindesten einem Strukturelement der obigen Formel (M1), gelten als bevorzugt:
- Vinylpyrrolidon/1-Vinyl-3-methyl-1H-imidazoliumchlorid-Copolymere (wie beispielsweise das mit der INCI-Bezeichnung Polyquaternium-16 unter den Handelsbezeichnungen Luviquat^{®} Style, Luviquat^{®} FC 370, Luviquat^{®} FC 550, Luviquat^{®} FC 905 und Luviquat^{®} HM 552 (BASF SE)),
- Vinylpyrrolidon/1-Vinyl-3-methyl-1H-imidazoliummethylsulfat-Copolymere (wie beispielsweise das mit der INCI-Bezeichnung Polyquaternium-44 unter den Handelsbezeichnungen Luviquat^{®} Care (BASF SE)),
- Vinylpyrrolidon/Vinylcaprolactam/1-Vinyl-3-methyl-1H-imidazolium-Terpolymer (wie beispielsweise das mit der INCI-Bezeichnung Polyquaternium-46 unter den Handelsbezeichnungen Luviquat^{®} Care oder Luviquat^{®} Hold (BASF SE)),
- Vinylpyrrolidon/Methacrylamid/Vinylimidazol/1-Vinyl-3-methyl-1H-imidazoliummethylsulfat-Copolymer (wie beispielsweise das mit der INCI-Bezeichnung Polyquaternium-68 unter der Handelsbezeichnung Luviquat^{®} Supreme (BASF SE)),
sowie Gemische aus diesen Polymeren.

Das erfindungsgemäße Mittel kann als zusätzliches filmbildendes und/oder festigendes Polymer mindestens ein filmbildendes nichtionisches und/oder festigendes nichtionisches Polymer enthalten. Unter einem nichtionischen Polymer wird erfindungsgemäß ein Polymer verstanden, das in einem protischen Lösemittel bei Standardbedingungen im Wesentlichen keine Struktureinheiten mit permanent kationischen oder anionischen Gruppen trägt, welche durch Gegenionen unter Erhaltung der Elektroneutralität kompensiert werden müssen. Unter kationische Gruppen fallen beispielsweise quaternisierte Ammoniumgruppen jedoch keine protonierten Amine. Unter anionische Gruppen fallen beispielsweise Carboxyl- und Sulfonsäuregruppen.

Die filmbildenden nichtionischen und/oder festigenden nichtionischen Polymere sind in dem erfindungsgemäßen Mittel dieser Ausführungsform bevorzugt in einer Menge von 0,1 Gew.-% bis 20,0 Gew.-%, besonders bevorzugt von 0,2 Gew.-% bis 15,0 Gew.-%, ganz besonders bevorzugt von 0,5 Gew.-% bis 10,0 Gew.-%, jeweils bezogen auf das Gewicht des erfindungsgemäßen Mittels, enthalten.

Es sind erfindungsgemäß solche filmbildende nichtionische und/oder festigende nichtionische Polymere mit mindestens einem Strukturelement der Formel (M2) 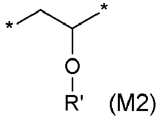 bevorzugt geeignet, welche gemäß Formel (M2) als R' ein Wasserstoffatom, eine Acetylgruppe oder eine Propanoylgruppe, insbesondere eine Acetylgruppe, tragen. Die filmbildenden nichtionischen und/oder festigenden nichtionischen Polymere werden wiederum bevorzugt ausgewählt aus mindestens einem Polymer der Gruppe, die gebildet wird, aus
- Homopolymeren und nichtionischen Copolymeren des N-Vinylpyrrolidons,
- nichtionischen Copolymeren des Isobutens.

Geeignete Polyvinylpyrrolidone sind beispielsweise Handelsprodukte wie Luviskol^{®} K 90 oder Luviskol^{®} K 85 der Firma BASF SE.

Geeignete Polyvinylalkohole werden beispielsweise unter den Handelsbezeichnungen Elvanol^{®} von Du Pont oder Vinol^{®} 523/540 von der Firma Air Products vertrieben.

Geeignetes Polyvinylacetat wird beispielsweise unter dem Handelsnamen Vinac^{®} als Emulsion von der Firma Air Products vertrieben.

Mittel, die als filmbildendes nichtionisches und/oder festigendes nichtionisches Polymer mindestens ein Polymer ausgewählt aus der Gruppe, die gebildet wird aus
- Polyvinylpyrrolidon,
- Copolymeren aus N-Vinylpyrrolidon und Vinylestern von Carbonsäuren mit 2 bis 18 Kohlenstoffatomen, insbesondere aus N-Vinylpyrrolidon und Vinylacetat,
- Copolymere aus N-Vinylpyrrolidon und N-Vinylimidazol und Methacrylamid,
- Copolymere aus N-Vinylpyrrolidon und N-Vinylimidazol und Acrylamid,
- Copolymere aus N-Vinylpyrrolidon mit N,N-Di(C₁ bis C₄)-Alkylamino-(C₂ bis C₄)-alkylacrylamid,
- Copolymere aus N-Vinylpyrrolidon mit N,N-Di(C₁ bis C₄)-Alkylamino-(C₂ bis C₄)-alkylacrylamid, sind erfindungsgemäß ganz besonders bevorzugt.

Weitere bevorzugte erfindungsgemäße Mittel dieser Ausführungsform sind dadurch gekennzeichnet, daß sie als nichtionisches filmbildendes und/oder nichtionisches festigendes Polymer mindestens ein Copolymer (c4) enthalten, das mindestens weitere Struktureinheit gemäß Formel (I) und mindestens eine Struktureinheit gemäß Formel (VII) und mindestens eine Struktureinheit gemäß Formel (VIII) enthält

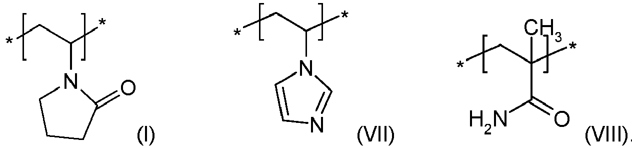

Auch hierbei ist besonders bevorzugt, wenn diese Copolymere neben Polymereinheiten, die aus dem Einbau der genannten Struktureinheiten gemäß Formel (M1-a), (I), (VII) und (VIII) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (c4) ausschließlich aus Struktureinheiten der Formel (M1-a), (I), (VII) und (VIII) aufgebaut und lassen sich durch die allgemeine Formel (Poly4) 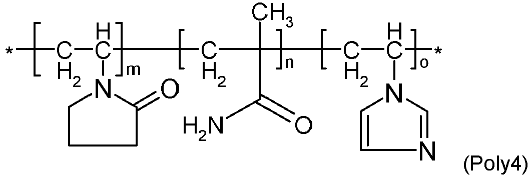 beschreiben, wobei die Indices m, n, o und p je nach Molmasse des Polymers variieren und nicht bedeuten sollen, daß es sich um Blockcopolymere handelt. Vielmehr können Struktureinheiten der Formeln (I), (VII) und (VIII) im Molekül statistisch verteilt vorliegen.

Ein besonders bevorzugtes Polymer wird dabei ausgewählt aus den Polymeren der INCI-Bezeichnung VP/Methacrylamide/Vinyl Imidazole Copolymer, die beispielsweise unter dem Handelsnamen Luviset Clear von der Fa. BASF SE erhältlich sind.

Als zusätzliche Co-Solventien können die erfindungsgemäßen Mittel organische Lösungsmittel oder ein Gemisch aus Lösungsmitteln mit einem Siedepunkt unter 400°C in einer Menge von 0,1 bis 15 Gewichtsprozent, bevorzugt von 1 bis 10 Gewichtsprozent bezogen auf das gesamte Mittel enthalten sein. Besonders geeignet als zusätzliche Co-Solventien sind unverzweigte oder verzweigte Kohlenwasserstoffe wie Pentan, Hexan, Isopentan und cyclische Kohlenwasserstoffe wie Cyclopentan und Cyclohexan. Weitere, besonders bevorzugte wasserlösliche Lösungsmittel sind Glycerin, Ethylenglykol und Propylenglykol in einer Menge bis 30 Gew.-% bezogen auf das gesamte Mittel.

Insbesondere der Zusatz von Glycerin und/oder Propylenglykol und/oder Polyethylenglykol und/oder Polypropylenglykol erhöht die Flexibilität des bei Anwendung des erfindungsgemäßen Mittels gebildeten Polymerfilms. Wird also ein flexibler Halt gewünscht, enthalten die erfindungsgemäßen Mittel vorzugsweise 0,01 bis 30 Gew.-% Glycerin und/oder Propylenglykol und/oder Polyethylenglykol und/oder Polypropylenglykol bezogen auf das gesamte Mittel.

Die Mittel weisen bevorzugt einen pH-Wert von 2 bis 11 auf. Besonders bevorzugt ist der pH-Bereich zwischen 2 und 8. Die Angaben zum pH-Wert beziehen sich dabei im Sinne dieser Schrift auf den pH-Wert bei 25°C, sofern nichts anderes vermerkt ist.

Die erfindungsgemäßen Mittel können weiterhin die Hilfs- und Zusatzstoffe enthalten, die üblicherweise herkömmlichen Stylingmitteln zugesetzt werden.

Als geeignete Hilfs- und Zusatzstoffe sind insbesondere zusätzliche Pflegestoffe zu nennen.

Als Pflegestoff kann das Mittel beispielsweise mindestens ein Proteinhydrolysat und/oder eines seiner Derivate enthalten.

Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L-Methionin-S-Methylsulfoniumchlorid. Selbstverständlich können erfindungsgemäß auch ß-Aminosäuren und deren Derivate wie ß-Alanin, Anthranilsäure oder Hippursäure eingesetzt werden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex), Sericin (Pentapharm) und Kerasol^{®} (Croda) vertrieben.

Besonders interessant ist der Einsatz von Seiden-Proteinhydrolysaten. Unter Seide versteht man die Fasern des Kokons des Maulbeer-Seidenspinners (Bombyx mori L.). Die Rohseidenfaser besteht aus einem Doppelfaden Fibroin. Als Kittsubstanz hält Sericin diesen Doppelfaden zusammen. Seide besteht zu 70 - 80 Gew.% aus Fibroin, 19 - 28 Gew.% Sericin, 0,5 - 1 Gew.% aus Fett und 0,5 - 1 Gew.% aus Farbstoffen und mineralischen Bestandteilen.

Die wesentlichen Bestandteile des Sericin sind mit ca. 46 Gew.% Hydroxyaminosäuren. Das Sericin besteht aus einer Gruppe von 5 bis 6 Proteinen. Die wesentlichen Aminosäuren des Sericines sind Serin (Ser, 37 Gew.%), Aspartat (Asp, 26 Gew.%), Glycin (Gly, 17 Gew.%), Alanin (Ala), Leucin (Leu) und Tyrosin (Tyr).

Das wasserunlösliche Fibroin ist zu den Skleroproteinen mit langkettiger Molekülstruktur zu zählen.

Die Hauptbestandteile des Fibroin sind Glycin (44 Gew.%), Alanin (26 Gew.%), und Tyrosin (13 Gew.%). Ein weiteres wesentliches Strukturmerkmal des Fibroins ist die Hexapeptidsequenz Ser-Gly-Ala-Gly-Ala-Gly.

Technisch ist es auf einfache Art und Weise möglich, die beiden Seidenproteine voneinander zu trennen. So verwundert es nicht, dass sowohl Sericin als auch Fibroin als Rohstoffe zur Verwendung in kosmetischen Produkten jeweils für sich allein bekannt sind. Weiterhin sind Proteinhydrolysate und -derivate auf der Basis der jeweils einzelnen Seidenproteine bekannte Rohstoffe in kosmetischen Mitteln. So wird beispielsweise Sericin als solches seitens der Fa. Pentapharm Ltd. als Handelsprodukt mit der Bezeichnung Sericin Code 303-02 vertrieben. Weitaus häufiger noch wird Fibroin als Proteinhydrolysat mit unterschiedlichen Molekulargewichten im Markt angeboten. Diese Hydrolysate werden insbesondere als "Seidenhydroylsate" vertrieben. So wird beispielsweise unter der Handelsbezeichnung Promois^{®} Silk hydrolysiertes Fibroin mit mittleren Molekulargewichten zwischen 350 und 1000 vertrieben.

Die positiven Eigenschaften der Seidenproteinderivate aus Sericin und Fibroin sind jeweils für sich genommen in der Literatur bekannt. So beschreibt die Verkaufsbroschüre der Fa. Pentapharm die kosmetischen Effekte des Sericines auf der Haut als reizlindernd, hydratisierend und filmbildend. Die Wirkung eines Fibroinderivates wird beispielsweise in der DE 31 39 438 A1 als pflegend und avivierend für das Haar beschrieben. Gemäß DE 102 40 757 A1 lässt sich bei einer gleichzeitigen Verwendung von Sericin und Fibroin bzw. deren Derivaten und/oder Hydrolysaten darüber hinaus eine synergistische Steigerung der positiven Wirkungen der Seidenproteine und deren Derivate erzielen.

Bevorzugt wird daher im erfindungsgemäßen Mittel als Seiden-Proteinhydrolysat ein Wirkstoffkomplex (A) bestehend aus dem Wirkstoff (A1) ausgewählt aus Sericin, Sericinhydrolysaten und/oder deren Derivaten, sowie Mischungen hieraus, und einem Wirkstoff (A2) ausgewählt aus Fibroin, und/oder Fibroinhydrolysaten und/oder deren Derivaten und/oder Mischungen hieraus eingesetzt.

Der Wirkstoffkomplex (A) verbessert signifikant in synergistischer Weise die zuvor dargestellten wesentlichen inneren und äußeren Strukturmerkmale und die Festigkeit sowie die Elastizität von menschlichen Haaren.

Besonders gute pflegende Eigenschaften können erzielt werden, wenn eine der beiden Wirkstoffkomponenten des Wirkstoffkomplexes (A) in der nativen oder allenfalls löslich gemachten Form verwendet wird. Es ist auch möglich, eine Mischung aus mehreren Wirkstoffen (A1) und/oder (A2) einzusetzen.

Es kann bevorzugt sein, dass die beiden Wirkstoffe (A1) und (A2) im Verhältnis von 10:90 bis 70:30, insbesondere 15:85 bis 50:50 und ganz besonders 20:80 bis 40:60, bezogen auf deren jeweilige Gehalte an aktiver Wirksubstanz in den erfindungsgemäßen Mitteln eingesetzt werden.

Proteinhydrolysate pflanzlichen Ursprungs, z. B. Soja-, Mandel-, Erbsen-, Kartoffel- und Weizenproteinhydrolysate, sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda) und Crotein^{®} (Croda) erhältlich.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon^{®} (Cognis), Lexein^{®} (Inolex), Crolastin^{®} (Croda), Crosilk^{®} (Croda) oder Crotein^{®} (Croda) vertrieben.

Selbstverständlich umfasst die erfindungsgemäße Lehre alle isomeren Formen, wie cis - trans-Isomere, Diastereomere und chirale Isomere. Erfindungsgemäß ist es auch möglich, eine Mischung aus mehreren Proteinhydrolysaten einzusetzen.

Die Proteinhydrolysate können in den erfindungsgemäßen Mitteln beispielsweise in Konzentrationen von 0,01 Gew.-% bis zu 20 Gew.-%, vorzugsweise von 0,05 Gew.-% bis zu 15 Gew.-% und ganz besonders bevorzugt in Mengen von 0,05 Gew.-% bis zu 5 Gew.-%, jeweils bezogen auf die gesamte Anwendungszubereitung enthalten sein.

Als Pflegestoff kann das erfindungsgemäße Mittel weiterhin mindestens ein Vitamin, ein Provitamin, eine Vitaminvorstufe und/oder eines derer Derivate enthalten.

Dabei sind erfindungsgemäß solche Vitamine, Provitamine und Vitaminvorstufen bevorzugt, die üblicherweise den Gruppen A, B, C, E, F und H zugeordnet werden.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die Mittel enthalten die Vitamin A-Komponente bevorzugt in Mengen von 0,05-1 Gew.-%, bezogen auf die gesamte Anwendungszubereitung.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäßen Mitteln bevorzugt in Mengen von 0,05 bis 1 Gew.-%, bezogen auf die gesamte Anwendungszubereitung, enthalten ist.
- Vitamin B₅ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie kationische Panthenolderivate. Die genannten Verbindungen des Vitamin B₅-Typs sind in den erfindungsgemäßen Mitteln bevorzugt in Mengen von 0,05 - 10 Gew.-%, bezogen auf die gesamte Anwendungszubereitung, enthalten. Mengen von 0,1 - 5 Gew.-% sind besonders bevorzugt.
- Vitamin B₆ (Pyridoxin sowie Pyridoxamin und Pyridoxal). Die genannten Verbindungen des Vitamin B₆-Typs sind in den erfindungsgemäßen Mitteln bevorzugt in Mengen von 0,01 - 5 Gew.-%, bezogen auf die gesamte Anwendungszubereitung, enthalten. Mengen von 0,05 - 1 Gew.-% sind besonders bevorzugt.

Vitamin C (Ascorbinsäure). Vitamin C wird in den erfindungsgemäßen Mitteln bevorzugt in Mengen von 0,1 bis 3 Gew.-%, bezogen auf die gesamte Anwendungszubereitung eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,05-1 Gew.-%, bezogen auf die gesamte Anwendungszubereitung, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3a*S*,4*S*, 6a*R*)-2-Oxohexahydrothienol[3,4-*d*]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.-%, insbesondere in Mengen von 0,001 bis 0,01 Gew.-%, jeweils bezogen auf die gesamte Anwendungszubereitung enthalten.

Bevorzugt enthalten die erfindungsgemäßen Mittel Vitamine, Provitamine und Vitaminvorstufen aus den Gruppen A, B, C, E und H.

Panthenol, Pantolacton, Pyridoxin und seine Derivate sowie Nicotinsäureamid und Biotin sind besonders bevorzugt.

Ganz besonders bevorzugt wird als Pflegestoff D-Panthenol, gegebenenfalls in Kombination mit mindestens einem der oben genannten Silikonderivate eingesetzt.

Wie auch der Zusatz von Glycerin und/oder Propylenglykol erhöht der Zusatz von Panthenol die Flexibilität des bei Anwendung des erfindungsgemäßen Mittels gebildeten Polymerfilms. Wird also ein besonders flexibler Halt gewünscht, können die erfindungsgemäßen Mittel statt oder zusätzlich zu Glycerin und/oder Propylenglykol Panthenol enthalten. In einer bevorzugten Ausführungsform enthalten die erfindungsgemäßen Mittel Panthenol, vorzugsweise in einer Menge von 0,05 bis 10 Gew.-%, besonders bevorzugt 0,1 - 5 Gew.-%, jeweils bezogen auf das gesamte Mittel.

Als Pflegestoff können die erfindungsgemäßen Mittel weiterhin mindestens einen Pflanzenextrakt enthalten.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß bevorzugten Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Grünem Tee, Eichenrinde, Brennnessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Besonders bevorzugt sind die Extrakte aus Grünem Tee, Eichenrinde, Brennnessel, Hamamelis, Hopfen, Kamille, Klettenwurzel, Schachtelhalm, Lindenblüten, Mandel, Aloe Vera, Kokosnuss, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel. Ganz besonders geeignet sind die Extrakte aus Grünem Tee, Mandel, Aloe Vera, Kokosnuss, Mango, Aprikose, Limone, Weizen, Kiwi und Melone.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt.

Pflanzenextrakte auf Basis von Wasser/Propylenglykol im Verhältnis 1:10 bis 10:1 haben sich als besonders geeignet erwiesen. Es ist erfindungsgemäß möglich, im Rahmen der vorgegebenen Wassermenge wasserhaltige Pflenzenextrakte einzusetzen. Dies ist jedoch erfindungsgemäß nicht bevorzugt.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Mitteln Mischungen aus mehreren, insbesondere aus zwei, verschiedenen Pflanzenextrakten einzusetzen.

Die erfindungsgemäßen Mittel enthalten diese Pflegestoffe bevorzugt in Mengen von 0,001 bis 2, insbesondere von 0,01 bis 0,5 Gew.-%, jeweils bezogen auf die gesamte Anwendungszubereitung. Auch Mono- bzw. Oligosaccharide können als Pflegestoff in den erfindungsgemäßen Mitteln eingesetzt werden.

Es können sowohl Monosaccharide als auch Oligosaccharide, wie beispielsweise Rohrzucker, Milchzucker und Raffinose, eingesetzt werden. Die Verwendung von Monosacchariden ist erfindungsgemäß bevorzugt. Unter den Monosacchariden sind wiederum solche Verbindungen bevorzugt, die 5 oder 6 Kohlenstoffatome enthalten.

Geeignete Pentosen und Hexosen sind beispielsweise Ribose, Arabinose, Xylose, Lyxose, Allose, Altrose, Glucose, Mannose, Gulose, Idose, Galactose, Talose, Fucose und Fructose. Arabinose, Glucose, Galactose und Fructose sind bevorzugt eingesetzte Kohlenhydrate; Ganz besonders bevorzugt eingesetzt wird Glucose, die sowohl in der D-(+)- oder L-(-)- Konfiguration oder als Racemat geeignet ist.

Weiterhin können auch Derivate dieser Pentosen und Hexosen, wie die entsprechenden Onsäuren, Uronsäuren, Zuckeralkohole und Glykoside, erfindungsgemäß eingesetzt werden. Bevorzugte Zuckersäuren sind die Gluconsäure, die Glucuronsäure, die Zuckersäure, die Mannozuckersäure und die Schleimsäure. Bevorzugte Zuckeralkohole sind Sorbit, Mannit und Dulcit. Bevorzugte Glykoside sind die Methylglucoside.

Da die eingesetzten Mono- bzw. Oligosaccharide üblicherweise aus natürlichen Rohstoffen wie Stärke gewonnen werden, weisen sie in der Regel die diesen Rohstoffen entsprechenden Konfigurationen auf (z.B. D-Glucose, D-Fructose und D-Galactose).

Die Mono- bzw. Oligosaccharide sind in den erfindungsgemäßen Mitteln bevorzugt in einer Menge von 0,1 bis 8 Gew.-%, insbesondere bevorzugt 1 bis 5 Gew.-%, bezogen auf die gesamte Anwendungszubereitung, enthalten.

Obwohl jeder der genannten Pflegestoffe für sich alleine bereits ein zufrieden stellendes Resultat ergibt, sind im Rahmen der vorliegenden Erfindung auch alle Ausführungsformen umfasst, in denen das Mittel mehrere Pflegestoffe auch aus verschiedenen Gruppen enthält.

Durch Zugabe eines UV-Filters können sowohl die Mittel selbst, als auch die behandelten Fasern vor schädlichen Einflüssen von UV-Strahlung geschützt werden. Vorzugsweise wird daher dem Mittel mindestens ein UV-Filter zugegeben. Die geeigneten UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die erfindungsgemäß bevorzugten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.

Bevorzugt sind solche UV-Filter, deren molarer Extinktionskoeffizient am Absorptionsmaximum oberhalb von 15 000, insbesondere oberhalb von 20 000, liegt.

Weiterhin wurde gefunden, dass bei strukturell ähnlichen UV-Filtern in vielen Fällen die wasserunlösliche Verbindung im Rahmen der erfindungsgemäßen Lehre die höhere Wirkung gegenüber solchen wasserlöslichen Verbindungen aufweist, die sich von ihr durch eine oder mehrere zusätzlich ionische Gruppen unterscheiden. Als wasserunlöslich sind im Rahmen der Erfindung solche UV-Filter zu verstehen, die sich bei 20 °C zu nicht mehr als 1 Gew.-%, insbesondere zu nicht mehr als 0,1 Gew.-%, in Wasser lösen. Weiterhin sollten diese Verbindungen in üblichen kosmetischen Ölkomponenten bei Raumtemperatur zu mindestens 0,1, insbesondere zu mindestens 1 Gew.-% löslich sein. Die Verwendung wasserunlöslicher UV-Filter kann daher erfindungsgemäß bevorzugt sein.

Die UV-Filter sind üblicherweise in Mengen von 0,01-5 Gew.-%, bezogen auf die gesamte Anwendungszubereitung, enthalten. Mengen von 0,1-2,5 Gew.-% sind bevorzugt.

In einer besonderen Ausführungsform enthält das erfindungsgemäße Mittel weiterhin einen oder mehrere direktziehende Farbstoffe. Dies ermöglicht, dass bei Anwendung des Mittels die behandelte keratinische Faser nicht nur temporär strukturiert, sondern zugleich auch gefärbt wird. Das kann insbesondere dann wünschenswert sein, wenn nur eine temporäre Färbung beispielsweise mit auffälligen Modefarben gewünscht wird, die sich durch einfaches Waschen wieder aus der keratinischen Faser entfernen lässt.

Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 11, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitro-phenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitro-diphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Bevorzugt werden kationische direktziehende Farbstoffe eingesetzt. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Ganz besonders bevorzugte kationische direktziehende Farbstoffe der Gruppe (c) sind die unter den Bezeichnungen Basic Yellow 87, Basic Orange 31 und Basic Red 51 bekannten Farbstoffe.

Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe.

Die erfindungsgemäßen Mittel gemäß dieser Ausführungsform enthalten die direktziehenden Farbstoffe bevorzugt in einer Menge von 0,001 bis 20 Gew.-%, bezogen auf das gesamte Mittel.

Weiterhin können die erfindungsgemäßen Mittel auch in der Natur vorkommende Farbstoffe enthalten, wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind.

Es ist nicht erforderlich, dass die direktziehenden Farbstoffe jeweils einheitliche Verbindungen darstellen. Vielmehr können in den erfindungsgemäßen Mitteln, bedingt durch die Herstellungsverfahren für die einzelnen Farbstoffe, in untergeordneten Mengen noch weitere Komponenten enthalten sein, soweit diese nicht das Stylingergebnis nachteilig beeinflussen oder aus anderen Gründen, z.B. toxikologischen, ausgeschlossen werden müssen.

Es ist erfindungsgemäß bevorzugt, dass die erfindungsgemäßen Mittel frei von Oxidationsfarbstoffvorprodukten sind. Oxidationsfarbstoffvorprodukte werden eingeteilt in sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus.

Die Mittel können neben den genannten Komponenten weiterhin alle für solche Zubereitungen bekannten Wirk-, Zusatz- und Hilfsstoffe enthalten.

Weitere Wirk-, Hilfs- und Zusatzstoffe, sind beispielsweise
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit, vollsynthetische Hydrokolloide wie z.B. Polyvinylalkohol, und gegebenenfalls vernetzte Polyacrylate,
- Strukturanten wie Maleinsäure und Milchsäure,
- Parfümöle, Dimethylisosorbid und Cyclodextrine,
- Entschäumer wie Silikone,
- Farbstoffe zum Anfärben des Mittels,
- Antischuppenwirkstoffe wie Piroctone Olamine, Zink Omadine und Climbazol,
- Substanzen zur Einstellung des pH-Wertes, wie beispielsweise übliche Säuren, insbesondere Genusssäuren, und Basen,
- Konsistenzgeber wie Zuckerester, Polyolester oder Polyolalkylether,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Konservierungsmittel,
- Antioxidantien.

Bezüglich weiterer fakultativer Komponenten sowie der eingesetzten Mengen dieser Komponenten wird ausdrücklich auf die dem Fachmann bekannten einschlägigen Handbücher verwiesen.

Die Formulierung der erfindungsgemäßen Mittel kann in allen für Stylingmittel üblichen Formen erfolgen, beispielsweise in Form von Lösungen, die als Lotion oder Pump- oder Aerosolspray auf das Haar aufgebracht werden können, oder anderen Zubereitungen, die für die Anwendung auf dem Haar geeignet sind.

Erfindungsgemäß liegen die erfindungsgemäßen Mittel in Form eines Aerosolsprays vor.

Hierzu werden die erfindungsgemäßen Mittel in einer Abgabevorrichtung konfektioniert, die ein zusätzlich mit einem Treibmittel befüllter Druckgasbehälter ("Aerosolbehälter") darstellt.

Die Druckgasbehälter, mit deren Hilfe ein Produkt durch den inneren Gasdruck des Behälters über ein Ventil verteilt wird, bezeichnet man definitionsgemäß als "Aerosolbehälter".

Erfindungsgemäß sind die erfindungsgemäßen Mittel als Aerosolhaarspray konfektioniert. Das erfindungsgemäße Mittel enthält daher zusätzlich mindestens ein Treibmittel.

Erfindungsgemäß geeignete Treibmittel sind beispielsweise ausgewählt aus N₂O, Dimethylether, CO₂, Luft, Alkanen mit 3 bis 5 Kohlenstoffatomen, wie Propan, n-Butan, iso-Butan, n-Pentan und isoPentan, und deren Mischungen. Bevorzugt sind Dimethylether, Propan, n-Butan, iso-Butan und Mischungen daraus. Gemäß einer bevorzugten Ausführungsform werden die genannten Alkane, Mischungen der genannten Alkane oder Mischungen der genannten Alkane mit Dimethylether als einziges Treibmittel eingesetzt. Die Erfindung umfasst aber ausdrücklich auch die Mitverwendung von Treibmitteln vom Typ der Fluorchlorkohlenwasserstoffe, insbesondere aber der Fluorkohlenwasserstoffe.

Die Menge an eingesetztem Treibmittel variiert in Abhängigkeit von der konkreten Zusammensetzung des Mittels, der verwendeten Verpackung und der gewünschten Produktart, etwa Haarspray oder Haarschaum. Das Treibmittel ist in den erfindungsgemäßen Mitteln bevorzugt in einer Menge von 30 bis 60 Gew.-% - bezogen auf das Gewicht des gesamten Mittels - enthalten.

Ganz besonders bevorzugt werden Mischungen von Propan und Butan als alleiniges Treibmittel verwendet im Gewichtsverhältnis Propan zu Butan von 20 zu 80 bis 15 zu 85. Diese Mischungen werden wiederum bevorzugt in den erfindungsgemäßen Mitteln in einer Menge von 30 bis 55 Gew.-% - bezogen auf das Gewicht des gesamten Mittels - eingesetzt. Unter Butan wird erfindungsgemäß n-Butan, iso-Butan und Gemische aus n-Butan und iso-Butan verstanden.

Über das Mengenverhältnis von Treibmittel zu den übrigen Bestandteilen der Zubereitungen lassen sich bei gegebener Sprühvorrichtung die Größen der Aerosoltröpfchen und die jeweilige Größenverteilung einstellen.

Die Sprührate der erfindungsgemäßen Sprays beträgt bevorzugt 6,5 bis 10,0 g/10 s.

Erfindungsgemäße Mittel in Form eines Aerosolsprays lassen sich in üblicher Art und Weise herstellen. In der Regel werden alle Bestandteile des erfindungsgemäßen Mittels mit Ausnahme des Treibmittels in einen geeigneten druckfesten Behälter eingefüllt. Dieser wird daraufhin mit einem Ventil verschlossen. Über herkömmliche Techniken wird schließlich die gewünschte Menge Treibmittel eingefüllt.

Besonders bevorzugte erfindungsgemäße Mittel sind in einem Aerosolbehälter mit einem Stem-Ventil mit einer Stembohrung mit 0,27 bis 0,35 mm Durchmesser konfektioniert. Solche Ventile werden beispielsweise als Ventile des Typs KE oder des Typs KEN von der Fa. Coster vertrieben. Darüberhinaus eignen sich erfindungsgemäß Düsen in der Ausgestaltung als sogenannte Wirbeldüsen besonders gut. Wirbeldüsen dieser Art werden beispielsweise unter der Bezeichnung "Standard micromist insert" von der Fa. Coster vertrieben. Wirbeldüsen weisen eine dem Fachmann bekannte Kanalführung zur Zentrumsmitte innerhalb der Düse auf.

Ein besonders bevorzugtes Aerosolprodukt dieser Erfindung ist ein in einem Aerosolbehälter konfektioniertes Mittel, enthaltend in einem kosmetisch akzeptablen Träger
(i) mindestens ein Copolymer (a) umfassend mindestens eine Struktureinheit der Formel (I) und mindestens eine Struktureinheit der Formel (II) und mindestens eine Struktureinheit der Formel (III) 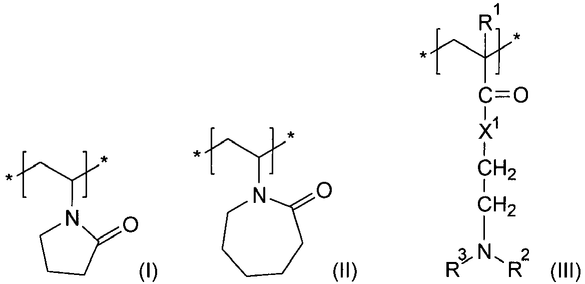 worin
   R¹ ein Wasserstoffatom oder eine Methylgruppe bedeutet,
   X¹ ein Sauerstoffatom oder eine Gruppe NH bedeutet,
   R² und R³ unabhängig voneinander für eine (C₁ bis C₄)-Alkylgruppe stehen,
(ii) mindestens ein Copolymer (b) umfassend mindestens eine Struktureinheit der Formel (I) und mindestens eine Struktureinheit der Formel (II) und mindestens eine Struktureinheit der Formel (IV) 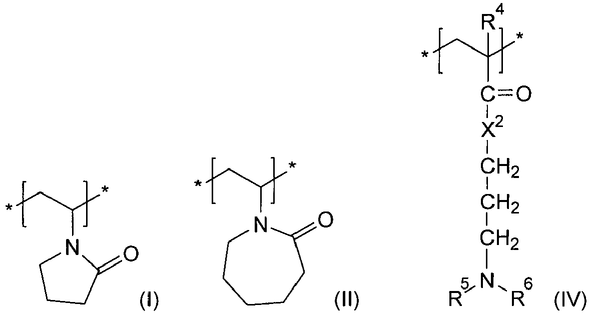 worin
   R⁴ ein Wasserstoffatom oder eine Methylgruppe bedeutet,
   X² ein Sauerstoffatom oder eine Gruppe NH bedeutet,
   R⁵ und R⁶ unabhängig voneinander für eine (C₁ bis C₄)-Alkylgruppe stehen
(iii) weniger als 1 Gew.-% Wasser
   und
(iv) mindestens ein Treibmittel, (insbesondere als alleiniges Treibmittel 30 bis 55 Gew.-% einer Mischung von Propan und Butan im Gewichtsverhältnis Propan zu Butan von 20 zu 80 bis 15 zu 85)
wobei der Aerosolbehälter ein Stem-Ventil mit einer Stembohrung mit 0,27 bis 0,35 mm Durchmesser aufweist und bevorzugt zusätzlich eine Wirbeldüse trägt und wobei es zusätzlich mindestens einen Alkohol, der 2 bis 6 Kohlenstoffatome und 1 bis 3 Hydroxylgruppen aufweist, enthält.

Hierbei eignen sich insbesondere die zuvor als bevorzugte gekennzeichneten Ausführungsformen der erfindungsgemäßen Inhaltsstoffe, die Angabe der bevorzugten Sprührate sowie deren Einsatzmengen als bevorzugt.

Ein ganz besonders bevorzugtes Aerosolprodukt dieser Erfindung ist daher ein in einem Aerosolbehälter konfektioniertes Mittel, enthaltend in einem kosmetisch akzeptablen Träger
(i) als Copolymer (a) mindestens ein Terpolymer aus N-Vinylpyrrolidon, N-Vinylcaprolactam und N,N-Dimethylaminoethylmethacrylat
   und
(ii) als Copolymer (b) mindestens ein Terpolymer aus N-Vinylpyrrolidon, N-Vinylcaprolactam und N,N-Dimethylaminopropylmethacrylamid
   und
(iii) weniger als 1 Gew.-% Wasser
   und
(iv) mindestens ein Treibmittel, (insbesondere als alleiniges Treibmittel 30 bis 55 Gew.-% einer Mischung von Propan und Butan im Gewichtsverhältnis Propan zu Butan von 20 zu 80 bis 15 zu 85)
wobei der Aerosolbehälter ein Stem-Ventil mit einer Stembohrung mit 0,27 bis 0,35 mm Durchmesser aufweist und bevorzugt zusätzlich eine Wirbeldüse trägt und
wobei es zusätzlich mindestens einen Alkohol, der 2 bis 6 Kohlenstoffatome und 1 bis 3 Hydroxylgruppen aufweist, enthält.

Die erfindungsgemäßen Aerosolsprays dieser Ausführungsformen besitzen bevorzugt eine Sprührate von 6,5 bis 10,0 g/10 s.

Ein zweiter Gegenstand der Erfindung ist ein Verfahren zur Behandlung keratinhaltiger Fasern, insbesondere menschlicher Haare, worin unter Einsatz einer Abgabevorrichtung ein Mittel gemäß erstem Erfindungsgegenstand als Aerosolspray auf die keratinhaltigen Fasern appliziert wird.

Dabei ist es erfindungsgemäß bevorzugt, dass die keratinhaltigen Fasern in Form gebracht werden und diese Form durch das Mittel des ersten Erfindungsgegenstandes fixiert wird.

Als erfindungsgemäß bevorzugt gelten die zuvor genannten Abgabevorrichtungen bzw. Aerosolprodukte (*vide supra*).

Die nachfolgenden Beispiele sollen den Gegenstand der vorliegenden Erfindung erläutern ohne ihn in irgendeiner Weise zu beschränken.

### Beispiele

Die folgenden Mengenangaben verstehen sich - soweit nichts anderes vermerkt ist - in Gewichtsprozent.

Folgende Rezepturen wurden durch Vermischen der angegebenen Rohstoffe bereitgestellt und in eine Aerosoldose mit einem Ventil der Fa. Coster, Typ KE und einer Wirbeldüse der Fa. Coster "Standard micromist insert", Typ V06 abgefüllt. Die Aerosoldosen wurden mit dem Ventil verschlossen und abschließend das entsprechende Treibmittel zugefügt:

| Rohstoffe | A | B | C |
|---|---|---|---|
| Ethanol, vergällt | 55,00 | 39,95 | 42,75 |
| Advantage LC-E ¹ | 6,50 | 6,50 | 4,50 |
| Aquaflex SF-40 ² | 3,00 | 3,00 | 2,00 |
| Neoheliopan E 1000 ³ | 0,10 | 0,10 | 0,20 |
| Triethylcitrat | 0,20 | 0,20 | - |
| Isopropylmyristat | - | - | 0,10 |
| Vitamin E Acetat | - | - | 0,20 |
| Parfum | 0,25 | 0,25 | 0,25 |
| Butan/Isobutan | 21,00 | - | - |
| Mischung aus 15% Propan 85% Butan/Isobutan | 14,00 | 50,00 | 50,00 |

| | | | |
|---|---|---|---|
| ¹ INCI-Bezeichnung: Vinylcaprolactam/VP/Dimethylaminoethylmethacrylate Copolymer, Laurylpyrrolidone; 37 Gew.-% Aktivsubstanz in Ethanol mit Zusatz von N-Laurylpyrrolidon (ISP) ² INCI-Bezeichnung: VP/Vinyl Caprolactam/DMAPA Acrylates Copolymer, Alcohol Denat.; 38-42 Gew.-% Aktivsubstanz in Ethanol (ISP) ³ 4-Methoxyzimtsäureisoamylester (INCI-Bezeichnung: Isoamyl-p-methoxycinnamate) (Symrise) | | | |

Alle resultierenden Aerosolsprays A, B und C wiesen eine Sprührate von 0,65-1,0 g/s auf. Nach Applikation auf das Haar bewirkten die erfindungsgemäßen Mittel einen hervorragenden, flexiblen Frisurenhalt. Das Haar erhielt einen natürlichen Glanz.

## Patentansprüche

1. Mittel, enthaltend in einem kosmetischen Träger
(i) mindestens ein Copolymer (a) umfassend mindestens eine Struktureinheit der Formel (I) und mindestens eine Struktureinheit der Formel (II) und mindestens eine Struktureinheit der Formel (III) 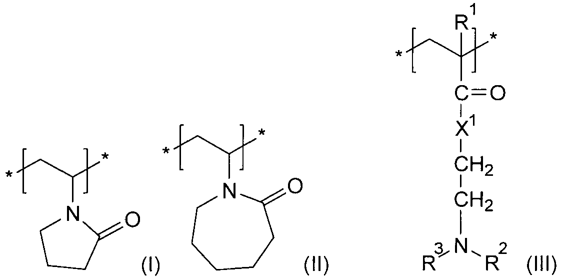 worin
R¹ ein Wasserstoffatom oder eine Methylgruppe bedeutet,
X¹ ein Sauerstoffatom oder eine Gruppe NH bedeutet,
R² und R³ unabhängig voneinander für eine (C₁ bis C₄)-Alkylgruppe stehen,
und
(ii) mindestens ein Copolymer (b) umfassend mindestens eine Struktureinheit der Formel (I) und mindestens eine Struktureinheit der Formel (II) und mindestens eine Struktureinheit der Formel (IV) 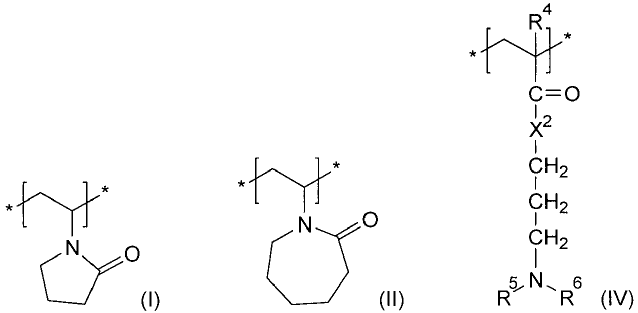 worin
R⁴ ein Wasserstoffatom oder eine Methylgruppe bedeutet,
X² ein Sauerstoffatom oder eine Gruppe NH bedeutet,
R⁵ und R⁶ unabhängig voneinander für eine (C₁ bis C₄)-Alkylgruppe stehen
und
(iii) weniger als 1 Gew.-% Wasser,
wobei es zusätzlich mindestens einen Alkohol, der 2 bis 6 Kohlenstoffatome und 1 bis 3 Hydroxylgruppen aufweist, enthält,
wobei es zusätzlich mindestens ein Treibmittel enthält und
wobei es in Form eines Aerosolsprays vorliegt.

2. Mittel nach Anspruch 1, **dadurch gekennzeichnet, dass** R¹ für eine Methylgruppe steht.

3. Mittel nach Anspruch 1 oder 2, **dadurch gekennzeichnet, dass** X¹ für ein Sauerstoffatom steht.

4. Mittel nach einem der Ansprüche 1 bis 3, **dadurch gekennzeichnet, dass** R² und R³ für eine Methylgruppe stehen.

5. Mittel nach einem der Ansprüche 1 bis 4, **dadurch gekennzeichnet, dass** R⁴ für eine Methylgruppe steht.

6. Mittel nach einem der Ansprüche 1 bis 5, **dadurch gekennzeichnet, dass** X² für eine Gruppe NH steht.

7. Mittel nach einem der Ansprüche 1 bis 6, **dadurch gekennzeichnet, dass** R⁵ und R⁶ für eine Methylgruppe stehen.

8. Mittel nach einem der Ansprüche 1 bis 7, **dadurch gekennzeichnet, dass** es das Copolymer (a) in einer Menge von 1,0 Gew.-% bis 5 Gew.-%, insbesondere von 1,5 Gew.-% bis 3,0 Gew.-%, jeweils bezogen auf das Gewicht des kosmetischen Mittels, enthält.

9. Mittel nach einem der Ansprüche 1 bis 8, **dadurch gekennzeichnet, dass** es das Copolymer (b) in einer Menge von 0,5 Gew.-% bis 2,5 Gew.-%, insbesondere von 0,7 Gew.-% bis 1,5 Gew.-%, jeweils bezogen auf das Gewicht des kosmetischen Mittels, enthält.

10. Mittel nach einem der Ansprüche 1 bis 9, **dadurch gekennzeichnet, dass** es das Copolymer (a) und das Copolymer (b) in einem Gewichtsverhältnisbereich (i) zu (ii) von 1 zu 1 bis 1 zu 5, insbesondere von 1 zu 1.5 bis 1 zu 3, enthält.

11. Mittel nach einem der vorangehenden Ansprüche, **dadurch gekennzeichnet, dass** der Alkohol, der 2 bis 6 Kohlenstoffatome und 1 bis 3 Hydroxylgruppen aufweist, ausgewählt wird aus mindestens einer Verbindung der Gruppe, die gebildet wird aus Ethanol, Isopropanol, 1,2-Propylenglykol, Glyzerin, n-Butanol, 1,3-Butylenglykol.

12. Verfahren zur Behandlung keratinhaltiger Fasern, insbesondere menschlicher Haare, worin unter Einsatz einer Abgabevorrichtung ein Mittel gemäß wenigstens eines der Ansprüche 1 bis 11 als Aerosolspray auf die keratinhaltigen Fasern appliziert wird.

## Claims

1. An agent which contains, in a cosmetic carrier:
(i) at least one copolymer (a) comprising at least one structural unit of formula (I) and at least one structural unit of formula (II) and at least one structural unit of formula (III) 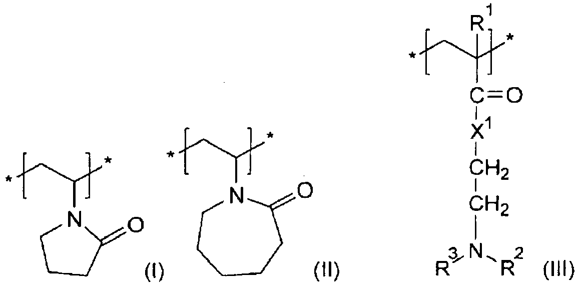 where
R¹ denotes a hydrogen atom or a methyl group,
X¹ denotes an oxygen atom or an NH group,
R² and R³, independently of one another, represent a (C₁ to C₄)-alkyl group, and
(ii) at least one copolymer (b) comprising at least one structural unit of formula (I) and at least one structural unit of formula (II) and at least one structural unit of formula (IV), 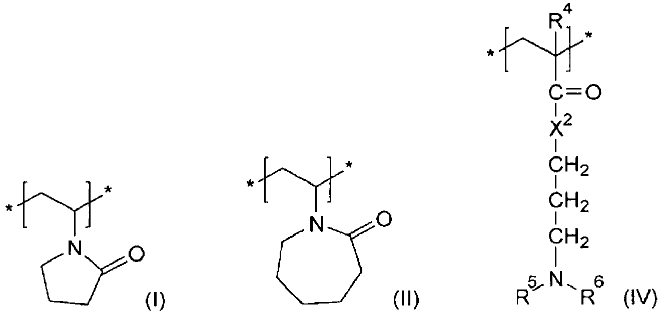 where
R⁴ denotes a hydrogen atom or a methyl group,
X² denotes an oxygen atom or a group NH,
R⁵ and R⁶, independently of one another, represent a (C₁ to C₄)-alkyl group
and
(iii) less than 1 wt.% water,
wherein it additionally contains at least one alcohol comprising from 2 to 6 carbon atoms and from 1 to 3 hydroxyl groups,
wherein it additionally contains at least one propellant and
wherein it is in the form of an aerosol spray.

2. The agent according to claim 1, **characterized in that** R¹ represents a methyl group.

3. The agent according to claim 1 or claim 2, **characterized in that** X¹ represents an oxygen atom.

4. The agent according to one of claims 1 to 3, **characterized in that** R² and R³ represent a methyl group.

5. The agent according to one of claims 1 to 4, **characterized in that** R⁴ represents a methyl group.

6. The agent according to one of claims 1 to 5, **characterized in that** X² represents an NH group.

7. The agent according to one of claims 1 to 6, **characterized in that** R⁵ and R⁶ represent a methyl group.

8. The agent according to one of claims 1 to 7, **characterized in that** it contains the copolymer (a) in an amount of from 1.0 wt.% to 5 wt.%, in particular from 1.5 wt.% to 3.0 wt.%, in each case based on the weight of the cosmetic agent.

9. The agent according to one of claims 1 to 8, **characterized in that** it contains the copolymer (b) in an amount of from 0.5 wt.% to 2.5 wt.%, in particular from 0.7 wt.% to 1.5 wt.%, in each case based on the weight of cosmetic agent.

10. The agent according to one of claims 1 to 9, **characterized in that** it contains the copolymer (a) and the copolymer (b) in a weight-ratio range (i):(ii) of from 1:1 to 1:5, in particular from 1:1.5 to 1:3.

11. The agent according to one of the preceding claims, **characterized in that** the alcohol, which comprises from 2 to 6 carbon atoms and from 1 to 3 hydroxyl groups, is selected from at least one compound of the group formed by ethanol, isopropanol, 1,2-propylene glycol, glycerol, n-butanol, 1,3-butylene glycol.

12. A method for treating keratin-containing fibers, in particular human hair, where, by using a dispensing device, an agent according to at least one of claims 1 to 11 is applied as an aerosol spray to the keratin-containing fibers.

## Revendications

1. Agent contenant, dans un support cosmétique
(i) au moins un copolymère (a) comprenant au moins une unité structurelle de la formule (I) et au moins une unité structurelle de la formule (II) et au moins une unité structurelle de la formule (III) 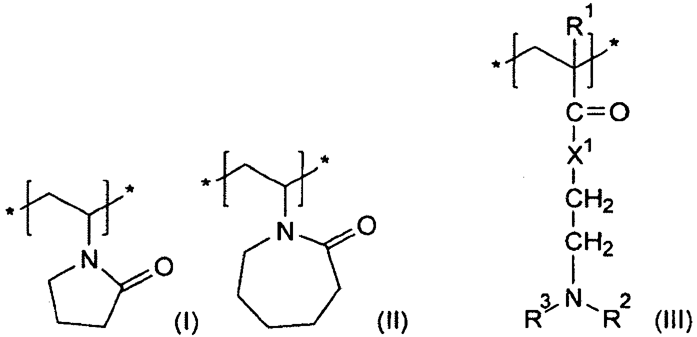 où
R¹ est un atome d'hydrogène ou un groupe méthyle,
X¹ représente un atome d'oxygène ou un groupe NH,
R² et R³ représentent indépendamment un groupe alkyle en C₁ à C₄,
et
(ii) au moins un copolymère (b) comprenant au moins une unité structurelle de la formule (I) et au moins une unité structurelle de la formule (II) et au moins une unité structurelle de la formule (IV) 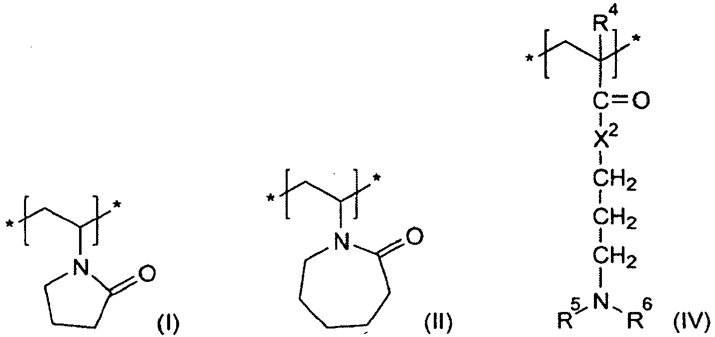 où
R⁴ représente un atome d'hydrogène ou un groupe méthyle,
X² représente un atome d'oxygène ou un groupe NH,
R⁵ et R⁶ représentent indépendamment un groupe alkyle en C₁ à C₄,
et
(iii) moins de 1% en poids d'eau,
l'agent contenant en outre au moins un alcool qui comporte de 2 à 6 atomes de carbone et de 1 à 3 groupes hydroxyle,
l'agent contenant en plus au moins un propulseur, et
l'agent se présentant sous la forme d'un spray aérosol.

2. Agent selon la revendication 1, **caractérisé en ce que** R¹ représente un groupe méthyle.

3. Agent selon la revendication 1 ou 2, **caractérisé en ce que** X¹ représente un atome d'oxygène.

4. Agent selon l'une des revendications 1 à 3, **caractérisé en ce que** R² et R³ représentent un groupe méthyle.

5. Agent selon l'une des revendications 1 à 4, **caractérisé en ce que** R⁴ représente un groupe méthyle.

6. Agent selon l'une des revendications 1 à 5, **caractérisé en ce que** X² représente un groupe NH.

7. Agent selon l'une des revendications 1 à 6, **caractérisé en ce que** R⁵ et R⁶ représentent un groupe méthyle.

8. Agent selon l'une des revendications 1 à 7, **caractérisé en ce qu'**il contient le copolymère (a) dans une quantité de 1,0% en poids à 5% en poids, en particulier de 1,5% en poids à 3,0% en poids, à chaque fois par rapport au poids de l'agent cosmétique.

9. Agent selon l'une des revendications 1 à 8, **caractérisé en ce qu'**il contient le copolymère (b) dans une quantité de 0,5% en poids à 2,5% en poids, en particulier de 0,7% en poids à 1,5% en poids, à chaque fois par rapport au poids de l'agent cosmétique.

10. Agent selon l'une des revendications 1 à 9, **caractérisé en ce qu'**il contient le copolymère (a) et le copolymère (b) dans une gamme de rapports en poids (i) sur (ii) de 1:1 à 1:5, en particulier de 1:1,5 à 1:3.

11. Agent selon l'une des revendications précédentes, **caractérisé en ce que** l'alcool, qui comporte 2 à 6 atomes de carbone et 1 à 3 groupes hydroxyle, choisis parmi au moins un composé du groupe formé par l'éthanol, l'isopropanol, le 1,2-propylène-glycol, le glycérol, le n-butanol, le 1,3-butylène-glycol.

12. Procédé de traitement de fibres de kératine, en particulier de cheveux humains, dans lequel un agent selon l'une au moins des revendications 1 à 11 est administré sous la forme d'un aérosol sur les fibres de kératine à l'aide d'un dispositif de distribution.
